# EUROPÄISCHE PATENTSCHRIFT

(11) **EP 0 982 291 B1**
(45) Veröffentlichungstag und Bekanntmachung des Hinweises auf die Patenterteilung: **26.03.2003**
(21) Anmeldenummer: 99113979.1
(22) Anmeldetag: 17.07.1999
(51) Int. Cl.: C07C 231/12, C07C 237/22, C07C 235/72

(54) **Verfahren zur Herstellung von Amidosäurephenylestern**
Process for the preparation of phenyl esters of amidocarboxylic acids
Procédé de préparation d'esters phényliques des acides amidocarboxyliques

(30) Priorität: 31.07.1998 DE 19834567; 18.12.1998 DE 19858660
(43) Veröffentlichungstag der Anmeldung: 01.03.2000
(73) Patentinhaber: Clariant GmbH, 65929 Frankfurt am Main (DE)
(72) Erfinder: Seebach, Michael, Dr., 65795 Hattersheim (DE); Naumann, Peter, Dr., 65232 Taunusstein (DE); Janitschek, Werner, 65779 Kelkheim (DE)

(56) Entgegenhaltungen:
- WO-A-96/39378
- US-A- 5 523 434

## Beschreibung

Die Erfindung betrifft die Synthese von Amidosäurephenylestern durch einstufige Umsetzung von Amidocarbonsäuren mit anorganischen Säurehalogeniden und einem Phenolderivat.

Amidosäurephenylester werden als Bleichaktivatoren in Wasch- und Reinigungsmitteln eingesetzt. Sie ermöglichen eine bleichende Wirkung bereits bei Temperaturen unter 60°C, indem sie mit einer Quelle für Wasserstoffperoxid - meist Perborate oder Percarbonate - unter Freisetzung einer organischen Peroxysäure reagieren.

In der Patentliteratur werden unterschiedliche Syntheseverfahren dieser Bleichaktivatoren beschrieben.
So beschreibt US-A-5 523 434 die Herstellung von Amidosäurephenylestern aus Amidocarbonsäuren und Phenolsulfonaten durch ein zweistufiges Verfahren: Im ersten Schritt erfolgt die Synthese eines Amidocarbonsäurechlorides durch Umsetzung der Amidocarbonsäure mit anorganischen Säurechloriden und in einem zweiten Schritt die Reaktion des Amidocarbonsäurechlorides mit einem Phenolsulfonat in einem Wasser/Diethylethergemisch. Problematisch für die großtechnische Anwendbarkeit dieses Verfahrens ist der Einsatz von Diethylether als Lösungsmittel, die geringe Lagerstabilität des Amidocarbonsäurechlorides sowie die Verwendung hoher Überschüsse an anorganischem Säurechlorid.

In US-A-5 466 840 wird ebenfalls ein mehrstufiges Syntheseverfahren von Amidosäurephenylestersulfonaten beschrieben. Darin wird das Alkalisalz einer 4-Hydroxybenzolsulfonsäure mit einem C₂-C₄-Carbonsäureanhydrid zum Alkalisalz einer 4-Acyloxybenzolsulfonsäure umgesetzt. Diese wird im zweiten Schritt durch Zugabe von 1-Oxyalkanoylaminocarbonsäure in Gegenwart eines Umesterungs-Katalysators bei 150 bis 250°C innerhalb von 0,5 bis 10 Stunden zum Amidosäurephenylestersulfonat überführt. Nachteil beider Synthesewege ist, daß mehrere Reaktionsstufen mit teils wenig stabilen Zwischenstufen durchlaufen werden müssen. Die Bildung von Nebenprodukten, Ausbeuteverluste und aufwendige Reinigungsverfahren der Produkte verteuern die Herstellung dieser als Bleichaktivatoren in Wasch- und Reinigungsmitteln einzusetzenden Stoffklasse.

In dem Verfahren gemäß WO 96/39378 werden Amidocarbonsäure und ein Phenolderivat in Sulfolan vorgelegt, ein Carbonsäureanhydrid, beispielsweise Acetanhydrid, zugetropft und durch Erhitzen auf ca. 170°C die Umsetzung zu Amidosäurephenylestersulfonaten je nach Ausgangsverbindung innerhalb von 0,5 bis 10 Stunden erreicht.

Unbefriedigend ist der sehr hohe Energieaufwand während der Reaktionsführung, verminderte Ausbeuten, stark verunreinigte Produkte sowie die sehr aufwendige und kostenintensive Abtrennung des hoch siedenden Lösungsmittels.
Es bestand daher die Aufgabe, eine verbesserte Verfahrensweise zur Herstellung von Amidosäurephenylestersulfonaten zu finden.

Gegenstand der Erfindung ist ein Verfahren zur Synthese von Amidosäurephenylestern der Formel I 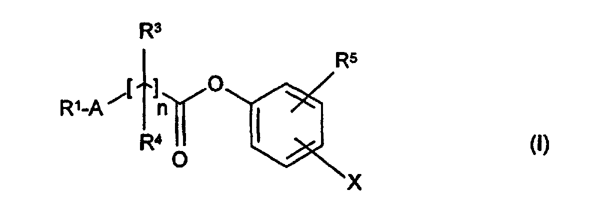 wobei
A eine Gruppe der Formel -CONR²- oder -NR²CO-,
R¹ eine Alkyl-, Alkenyl-, Alkinyl- oder Cycloalkylgruppe mit jeweils 1 bis 26 C-Atomen oder eine Aryl- oder Alkylarylgruppe mit jeweils 6 bis 14 C-Atomen,
R² Wasserstoff oder eine Alkyl-, Alkenyl-, Alkinyl- oder eine Cycloalkylgruppe mit jeweils 1 bis 26 C-Atomen oder eine Aryl- oder Alkylarylgruppe mit jeweils 6 bis 14 C-Atomen,
R³ und R⁴ die gleich oder verschieden sein können, jeweils Wasserstoff oder eine Alkyl-, Alkenyl-, Alkinyl- oder eine Cycloalkylgruppe mit jeweils 1 bis 10 C-Atomen,
R⁵ Wasserstoff, Halogen oder eine Alkyl-, Alkenyl-, Alkinyl-, Cycloalkyl- oder eine Alkoxygruppe mit jeweils 1 bis 6 C-Atomen bedeuten, n für eine Zahl von 1 bis 10 steht,
X eine Gruppe der Formeln SO₃M, OSO₃M, (CH₂)ₘSO₃M, (CH₂)ₘ-OSO₃M, CO₂M und N(R⁶)₃Y bedeutet,
wobei M für Wasserstoff oder ein Alkaliion,
R⁶ eine Alkylgruppe mit 1 bis 6 C-Atomen oder eine Cycloalkylgruppe mit 4 bis 6 C-Atomen,
Y ein Halogenatom und
m 1 oder 2 steht.

Bevorzugt ist die Herstellung der Verbindungen der Formel (I) wobei gleichzeitig A eine Gruppe der Formel -CONR²-, R¹ C₈-C₁₀-Alkyl, R², R³, R⁴ und R⁵ Wasserstoff, n = 5 und X -SO₃M bedeuten.

Dieses Verfahren besteht darin, daß man ein anorganisches Säurehalogenid zu einer Mischung der Verbindungen der Formeln II und III 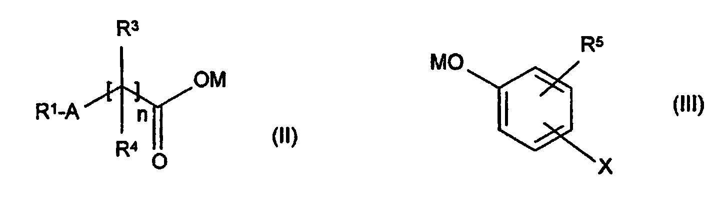 gibt, wobei A, R¹, R², R³, R⁴, R⁵ und M die zuvor angegebenen Bedeutungen haben.

Für das erfindungsgemäße Verfahren geht man im einzelnen so vor, daß man zunächst die beiden Ausgangsverbindungen der Formeln II bzw. III gemeinsam in einem geeigneten organischen Lösemittel löst oder suspendiert. Als Lösemittel kommen beispielsweise infrage Xylol, Benzol, Monoglyme, Diglyme, Diisopropylether, Tetrahydrofuran, Dioxan, Isobutylmethylketon, Aceton, Diethylketon, Acetonitril, Carbonsäurealkylester oder deren Gemische. Beispiele für Carbonsäurealkylester sind Essigsäure-C₂-C₄-alkylester, wie Essigsäure-n-propylester, Essigsäure-i-propylester, Essigsäure-n-butylester, Essigsäure-i-butylester, Essigsäure-t-butylester oder deren Gemische. Bevorzugt ist Toluol oder n-Butylacetat. Das molare Verhältnis der Verbindungen der Formeln II und III beträgt 1:0,7 bis 1,5, bevorzugt 1:0,8 bis 1,3.

Zu dieser Lösung bzw. Suspension der Verbindungen II und III wird ein anorganisches Säurehalogenid zugetropft, beispielsweise PCl₃, PCl₅, POCl₃, COCl₂, bevorzugt SOCl₂. Anstelle dieser Chloride können auch die analogen Bromide eingesetzt werden. Die Menge an Säurehalogenid beträgt 0,5 bis 2, bevorzugt 0,7 bis 1,5, insbesondere 0,9 bis 1,4 Moläquivalente bezogen auf die Amidocarbonsäure. Die Temperatur, bei der die Reaktion durchgeführt wird, liegt im allgemeinen bei 25 bis 120°C, bevorzugt bei 50 bis 110°C, besonders bevorzugt bei 60 bis 100°C.

Die Reaktion läuft in einem Zeitraum von 10 Minuten bis 8 Stunden, bevorzugt 30 Minuten bis 5 Stunden ab, dem sich eine Nachrührzeit von 10 Minuten bis 5 Stunden, bevorzugt 30 Minuten bis 3 Stunden, anschließt.

Das verwendete Lösungsmittel kann nach Beendigung der Umsetzung durch Destillation, Abdekantieren oder Abhebern vom Reaktionsprodukt entfernt werden. Die Reaktionsmischung wird nach Verdünnen mit Wasser durch Zugabe einer Base, bevorzugt Natronlauge, Kalilauge, Natriumcarbonat oder Kaliumcarbonat auf einen pH-Wert von pH 4 bis pH 11, bevorzugt pH 7 bis pH 10 eingestellt. Die dabei erhaltenen Endprodukte können durch Filtration, Abnutschen, Abdekantieren oder durch Zentrifugation aus dieser Mutterlauge abgetrennt werden. Zur Reinigung kann das feuchte Produkt mit Wasser, Alkoholen, aromatischen Lösungsmitteln, Alkanen, Ketonen oder Estem, sowie Gemischen aus diesen, vorzugsweise mit Wasser, Alkoholen oder Estern oder deren Gemischen ausgerührt oder umkristallisiert werden.

Nach dem erfindungsgemäßen Syntheseverfahren wird die Zielverbindung in Ausbeuten von über 80 % und hoher Reinheit (Gehalt an Amidosäurephenylester über 90 %) erhalten.

Nachfolgende Beispiele sollen die Erfindung näher erläutern ohne sie darauf einzuschränken.

### Beispiele

Die Beispiele 1 bis 7 erläutern die Synthese von n-Nonanoylamidocaproyl-oxy-benzolsulfonsäure-Natriumsalz unter Verwendung unterschiedlicher Lösungsmittel und Bedingungen bei der Aufarbeitung.
1. Synthese von n-Nonanoylamidocaproyl-oxy-benzolsulfonsäure-Natriumsalz in Toluol
   135,7 g (0,5 mol) n-Nonanoylamidohexansäure und 100,1 g (0,5 mol) p-Phenolsulfonsäure-Natriumsalz (98 %) wurden in 600 ml Toluol suspendiert und auf 80°C erhitzt Zu dieser Mischung wurden innerhalb von 3 Stunden 77,4 g (0,65 mol) Thionylchlorid getropft und 2 Stunden bei 80°C nachgerührt. Danach wurden am Vakuum bei 30 bis 40°C etwa 250 ml Toluol abdestilliert und 1000 ml Wasser unter starkem Rühren zugegeben. Durch Zutropfen von 78 g Natriumhydroxidlösung (32 %) wurde ein pH-Wert von 8,0 bis 8,5 eingestellt. Das ausgefallene Produkt wurde abgesaugt, zweimal mit je 100 ml Wasser gewaschen und über Nacht bei 50°C im Vakuumtrockenschrank getrocknet. Es wurden 189,2 g (84 %) n-Nonanoylamidocaproyl-oxy-benzolsulfonsäure-Natriumsalz als beigeweißer Feststoff erhalten. Die folgenden Analysendaten des Produktes wurden mittels HPLC bestimmt:
   94,0 % n-Nonanoylamidocaproyl-oxy-benzolsulfonsäure-Natriumsalz
   0,9 % n-Nonanoylamidohexansäure
   0,4 % p-Phenolsulfonsäure-Natriumsalz
   0,7 % Nonanoyloxy-benzolsulfonsäure-Natriumsalz
2. Synthese von n-Nonanoylamidocaproyl-oxy-benzolsulfonsäure-Natriumsalz in Toluol ohne Abdestillieren des Lösungsmittels
   135,7 g (0,5 mol) n-Nonanoylamidohexansäure und 100,1 g (0,5 mol) p-Phenolsulfonsäure-Natriumsalz (98 %) wurden in 750 ml Toluol suspendiert und auf 80°C erhitzt. Zu dieser Mischung wurden innerhalb von 4 Stunden 77,4 g (0,65 mol) Thionylchlorid getropft und 2 Stunden bei 80°C nachgerührt. Danach wurde die Toluolphase durch Abdekantieren und Abhebem weitgehend abgetrennt und der Rückstand mit 500 ml Wasser verdünnt. Durch Zutropfen von 119 g Natriumhydroxidlösung (32 %) wurde ein pH-Wert von 9,0 eingestellt. Das ausgefallene Produkt wurde abgesaugt, zweimal mit je 100 ml Wasser nachgewaschen und über Nacht bei 50°C im Vakuumtrockenschrank getrocknet. Es wurden 192,0 g (85,4 %) n-Nonanoylamidocaproyl-oxy-benzolsulfonsäure-Natriumsalz als weißer Feststoff erhalten. Die folgenden Analysendaten des Produktes wurden mittels HPLC bestimmt:
   93,5 % n-Nonanoylamidocaproyl-oxy-benzolsulfonsäure-Natriumsalz
   1,6 % n-Nonanoylamidohexansäure
   0,2 % p-Phenolsulfonsäure-Natriumsalz
   0,7 % Nonanoyloxy-benzolsulfonsäure-Natriumsalz
3. Synthese von n-Nonanoylamidocaproyl-oxy-benzolsulfonsäure-Natriumsalz in Di-isopropylether
   67,85 g (0,25 mol) n-Nonanoylamidohexansäure und 50,05 g (0,25 mol) p-Phenolsulfonsäure-Natriumsalz (98 %) wurden in 370 ml Diisopropylether suspendiert und auf 69°C erhitzt. Zu dieser Mischung wurden unter Rückfluß innerhalb von 3 Stunden 37,2 g (0,313 mol) Thionylchlorid getropft sowie 2 Stunden bei Rückfluß nachgerührt. Danach wurden am Vakuum bei 30 bis 40°C etwa 200 ml Ether abdestilliert und 750 ml Wasser unter starkem Rühren zugegeben. Durch Zutropfen von 52 g Natriumhydroxidlösung (32 %) wurde ein pH-Wert von 8,6 eingestellt. Das ausgefallene Produkt wurde abgesaugt, zweimal mit je 50 ml Wasser gewaschen und über Nacht bei 50°C im Vakuumtrockenschrank getrocknet. Es wurden 98,7 g (88 %) n-Nonanoylamidocaproyl-oxy-benzolsulfonsäure-Natriumsalz als beige-weißer Feststoff erhalten. Die folgenden Analysendaten des Produktes wurden mittels HPLC bestimmt:
   91,7 % n-Nonanoylamidocaproyl-oxy-benzolsulfonsäure-Natriumsalz
   1,2 % n-Nonanoylamidohexansäure
   0,1% p-Phenolsulfonsäure-Natriumsalz
   0,8 % Nonanoyloxy-benzolsulfonsäure-Natriumsalz
4. Synthese von n-Nonanoylamidocaproyl-oxy-benzolsulfonsäure-Natriumsalz in Toluol und anschließende Aufarbeitung durch Ausrühren in Wasser
   67,85 g (0,25 mol) n-Nonanoylamidohexansäure und 50,05 g (0,25 mol) p-Phenolsulfonsäure-Natriumsalz (98 %) wurden in 370 ml Toluol suspendiert und auf 75°C erhitzt. Zu dieser Mischung wurden unter Rückfluß innerhalb von 3 Stunden 37,2 g (0,313 mol) Thionylchlorid getropft sowie 2 Stunden bei Rückfluß nachgerührt. Danach wurden am Vakuum bei 30°C etwa 117 ml Toluol abdestilliert und 750 ml Wasser zugegeben. Durch Zutropfen von 41,3 g Natriumhydroxidlösung (32 %) wurde ein pH-Wert von 8,5 eingestellt. Nach 30 Minuten Nachrühren wurde das ausgefallene Produkt abgesaugt und eine Stunde mit 300 ml Wasser bei 40°C ausgerührt. Nach dem Abkühlen wurde abermals abgesaugt und das Produkt über Nacht bei 50°C im Vakuumtrockenschrank getrocknet. Es wurden 93,1 g (83 %) n-Nonanoylamidocaproyl-oxy-benzolsulfonsäure-Natriumsalz als weißer Feststoff erhalten. Die folgenden Analysendaten des Produktes wurden mittels HPLC bestimmt:
   98,4 % n-Nonanoylamidocaproyl-oxy-benzolsulfonsäure-Natriumsalz
   0,3 % n-Nonanoylamidohexansäure
   0,1 % p-Phenolsulfonsäure-Natriumsalz
   0,1 % Nonanoyloxy-benzolsulfonsäure-Natriumsalz
   Der Vergleich mit den Analysedaten aus Beispiel 1 belegt, daß durch Ausrühren ein zusätzlicher Reinigungseffekt ohne signifikante Ausbeuteverluste erzielt werden kann.
5. Synthese von n-Nonanoylamidocaproyl-oxy-benzolsulfonsäure-Natriumsalz in n-Butylacetat
   135,7 g (0,5 mol) n-Nonanoylamidohexansäure und 100,1 g (0,5 mol) p-Phenolsulfonsäure-Natriumsalz (98 %) wurden in 600 ml Toluol suspendiert und unter N₂-Atmosphäre auf 90°C erhitzt. Zu dieser Mischung wurden innerhalb von 3 Stunden 77,4 g (0,65 mol) Thionylchlorid getropft und 1 Stunde bei 90°C nachgerührt. Nach dem Abkühlen auf 30°C wurden 900 ml Wasser unter starkem Rühren zugegeben. Durch Zutropfen von 128 g Natriumhydroxidlösung (32 %) bei einer Temperatur von 35 bis 40°C wurde ein pH-Wert von 8,0 eingestellt. Das ausgefallene Produkt wurde auf 20°C abgekühlt und abgesaugt, einmal mit 100 ml Wasser gewaschen und über Nacht bei 60°C im Vakuumtrockenschrank getrocknet.
   Es wurden 198,6 g (88,3 %) n-Nonanoylamidocaproyl-oxy-benzolsulfonsäure-Natriumsalz als beige-weißer Feststoff erhalten. Die folgenden Analysendaten des Produktes wurden mittels HPLC bestimmt:
   92,7 % n-Nonanoylamidocaproyl-oxy-benzolsulfonsäure-Natriumsalz
   0,7 % n-Nonanoylamidohexansäure
   0,7 % p-Phenolsulfonsäure-Natriumsalz
   0,3 % Nonanoyloxy-benzolsulfonsäure-Natriumsalz.
6. Synthese von n-Nonanoylamidocaproyl-oxy-benzolsulfonsäure-Natriumsalz in iso-Propylacetat
   135,7 g n-Nonanoylamidohexansäure und 101,1 g p-Phenolsulfonsäure-Natriumsalz (98 %) wurden in 300 ml iso-Propylacetat suspendiert und auf 80°C erhitzt. Zu dieser Mischung wurden innerhalb von 3 Stunden 77,4 g Thionylchlorid getropft und 1 Stunde bei 80°C nachgerührt. Nach dem Abkühlen auf Raumtemperatur wurden 700 ml Wasser zugegeben und durch Zutropfen von 109 g Natriumhydroxidlösung (32 %) ein pH-Wert von 7,5 bis 8,0 eingestellt. Das ausgefallene Produkt wurde abgesaugt und über Nacht bei 60°C im Vakuumtrockenschrank getrocknet. Es wurden 200,0 g (89 %) n-Nonanoylamidocaproyl-oxy-benzolsulfonsäure-Natriumsalz als weißer Feststoff erhalten. Die folgenden Analysendaten des Produktes wurden mittels HPLC bestimmt:
   92,3 % n-Nonanoylamidocaproyl-oxy-benzolsulfonsäure-Natriumsalz
   1,1% n-Nonanoylamidohexansäure
   0,4 % p-Phenolsulfonsäure-Natriumsalz
   1,0 % Nonanoyloxy-benzolsulfonsäure-Natriumsalz
7. Synthese von n-Nonanoylamidocaproyl-oxy-benzolsulfonsäure-Na-Salz in iso-Butylacetat
   135,7 g n-Nonanoylamidohexansäure und 100,9 g p-Phenolsulfonsäure-Natriumsalz (98 %) wurden in 300 ml iso-Butylacetat suspendiert und auf 90 bis 95°C erhitzt Zu dieser Mischung wurden innerhalb von 3 Stunden 77,6 g Thionylchlorid getropft und 1 Stunde bei 90°C nachgerührt. Nach dem Abkühlen auf 20°C wurden 700 ml Wasser zugegeben und bei einer Temperatur von 35 - 40°C durch Zutropfen von 118 g Natriumhydroxidlösung (32 %) ein pH-Wert von 8,0 eingestellt. Das ausgefallene Produkt wurde bei 20°C abgesaugt und über Nacht bei 60°C im Vakuumtrockenschrank getrocknet. Es wurden 200,3 g (89 %) n-Nonanoylamidocaproyl-oxy-benzolsulfonsäure-Natriumsalz als weißer Feststoff erhalten. Die folgenden Analysendaten des Produktes wurden mittels HPLC bestimmt:
   93,9 % n-Nonanoylamidocaproyl-oxy-benzolsulfonsäure-Natriumsalz
   0,7 % n-Nonanoylamidohexansäure
   0,5 % p-Phenolsulfonsäure-Natriumsalz
   0,4 % Nonanoyloxy-benzolsulfonsäure-Natriumsalz

   Die Beispiele 8 bis 10 beschreiben die Synthese von n-Decanoylamidocaproyl-oxybenzolsulfonsäure-Natriumsalz unter Verwendung unterschiedlicher Lösungsmittel und verschiedener Bedingungen bei der Aufarbeitung.
8. Synthese von n-Decanoylamidocaproyl-oxy-benzolsulfonsäure-Natriumsalz in Toluol
   142,7 g (0,5 mol) n-Decanoylamidohexansäure und 98,1 g (0,5 mol) p-Phenolsulfonsäure-Natriumsalz (100 %) wurden in 400 ml Toluol suspendiert und auf 80°C erhitzt. Zu dieser Mischung wurden innerhalb von 4 Stunden 62,5 g (0,525 mol) Thionylchlorid getropft und 2 Stunden bei 80°C nachgerührt. Danach wurden 15,0 g Natriumhydroxid-Microprills zugesetzt und beim Nachrühren mit Toluol verdünnt. Das abgesaugte Reaktionsprodukt wurde in 500 ml Wasser eingerührt und der pH-Wert auf 7,5 eingestellt. Nach abermaligem Absaugen wurde zweimal mit jeweils 150 ml Wasser gewaschen und über Nacht bei 50°C im Vakuumtrockenschrank getrocknet. Es wurden 220,3 g (95 %) n-Decanoylamidocaproyl-oxy-benzolsulfonsäure-Natriumsalz als weißer Feststoff erhalten. Die folgenden Analysendaten des Produktes wurden mittels HPLC bestimmt:
   93,6 % n-Decanoylamidocaproyl-oxy-benzolsulfonsäure-Natriumsalz
   0,1 % p-Phenolsulfonsäure-Natriumsalz
   1,3 % Decanoyloxy-benzolsulfonsäure-Natriumsalz
9. Synthese von n-Decanoylamidocaproyl-oxy-benzolsulfonsäure-Natriumsalz in Diethylenglykol-dimethylether
   74,1 g (0,25 mol) n-Decanoylamidohexansäure und 49,1 g (0,25 mol) p-Phenolsulfonsäure-Natriumsalz (100 %) wurden in 375 ml Diethylenglykol-dimethylether vorgelegt. Hierzu wurden bei 80°C innerhalb von 3 Stunden 32,7 g (0,275 mol) Thionylchlorid zugetropft und bei gleicher Temperatur 3 Stunden nachgerührt. Das Reaktionsprodukt wurde bei Raumtemperatur abgesaugt, mit 100 ml Diethylenglykol-dimethylether gewaschen und bei 30°C im Vakuum getrocknet. Das Rohprodukt wurde in 250 ml Wasser mit 20 g Natronlauge (33 %) neutralisiert, abermals abgesaugt, mit dreimal 50 ml Wasser gewaschen und bei 30°C im Vakuum getrocknet. Es wurden 84,4 g (73 %) n-Decanoylamidocaproyl-oxy-benzolsulfonsäure-Natriumsalz als beige-weißer Feststoff erhalten. Die folgenden Analysendaten des Produktes wurden mittels HPLC bestimmt:
   94,7 % n-Decanoylamidocaproyl-oxy-benzolsulfonsäure-Natriumsalz
   0,2 % p-Phenolsulfonsäure-Natriumsalz
   0,3 % Decanoyloxy-benzolsulfonsäure-Natriumsalz
10. Synthese von n-Decanoylamidocaproyl-oxy-benzolsulfonsäure-Natriumsalz in Toluol unter Verwendung von Natriumcarbonat zur Neutralisation
   142,7 g (0,5 mol) n-Decanoylamidohexansäure und 98,1 g (0,5 mol) p-Phenolsulfonsäure-Natriumsalz (100 %) wurden in 400 ml Toluol vorgelegt und auf 80°C erhitzt. Zu dieser Mischung wurden innerhalb von 4 Stunden 60,7 g (0,51 mol) Thionylchlorid getropft und 2 Stunden bei 80°C nachgerührt. Danach wurden 10,6 g Natriumcarbonat zugegeben und 30 Minuten nachgerührt. Das Reaktionsgemisch wurde abgesaugt und in 500 ml Wasser eingerührt. Mit Natronlauge (33 %) wurde ein pH-Wert auf 7,5 eingestellt und abermals abgesaugt. Nach zweimaligem Waschen mit jeweils 150 ml Wasser und Trocknen des Rückstandes im Vakuumtrockenschrank wurden 221,4 g (96 %) n-Decanoylamidocaproyl-oxy-benzolsulfonsäure-Natriumsalz als beige-weißer Feststoff erhalten. Die folgenden Analysendaten des Produktes wurden mittels HPLC bestimmt:
   92,8 % n-Decanoylamidocaproyl-oxy-benzolsulfonsäure-Natriumsalz
   0,3 % p-Phenolsulfonsäure-Natriumsalz
   1,5 % Decanoyloxy-benzolsulfonsäure-Natriumsalz

## Patentansprüche

1. Verfahren zur Herstellung von Amidosäurephenylestern der Formel I 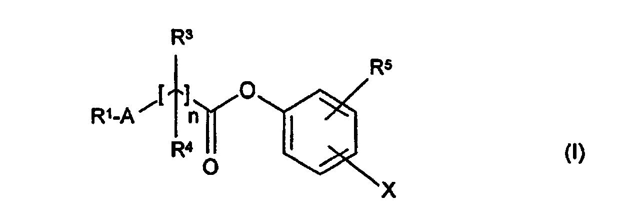 wobei
A eine Gruppe der Formel -CONR²- oder -NR²CO-,
R¹ eine Alkyl-, Alkenyl-, Alkinyl- oder Cycloalkylgruppe mit jeweils 1 bis 26 C-Atomen oder eine Aryl- oder Alkylarylgruppe mit jeweils 6 bis 14 C-Atomen,
R² Wasserstoff oder eine Alkyl-, Alkenyl-, Alkinyl- oder eine Cycloalkylgruppe mit jeweils 1 bis 26 C-Atomen oder eine Aryl- oder Alkylarylgruppe mit jeweils 6 bis 14 C-Atomen,
R³ und R⁴ die gleich oder verschieden sein können, jeweils Wasserstoff oder eine Alkyl-, Alkenyl-, Alkinyl- oder eine Cycloalkylgruppe mit jeweils 1 bis 10 C-Atomen,
R⁵ Wasserstoff, Halogen oder eine Alkyl-, Alkenyl-, Alkinyl-, Cycloalkyl- oder eine Alkoxygruppe mit jeweils 1 bis 6 C-Atomen bedeuten, n für eine Zahl von 1 bis 10 steht,
X eine Gruppe der Formeln SO₃M, OSO₃M, (CH₂)ₘSO₃M, (CH₂)ₘO-SO₃M, CO₂M und N(R⁶)₃Y bedeutet,
wobei M für Wasserstoff oder ein Alkaliion,
R⁶ eine Alkylgruppe mit 1 bis 6 C-Atomen oder eine Cycloalkylgruppe mit 4 bis 6 C-Atomen,
Y ein Halogenatom und
m für 1 oder 2 steht,
**dadurch gekennzeichnet, daß** man ein anorganisches Säurehalogenid zu einer Mischung der Verbindungen der Formeln II und III 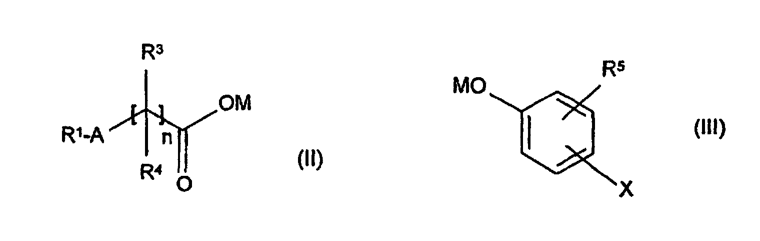 gibt, wobei A, R¹, R², R³, R⁴, R⁵, X und M die zuvor angegebenen Bedeutungen haben.

2. Verfahren nach Anspruch 1, **dadurch gekennzeichnet, daß** man mit einem molaren Verhältnis der Verbindungen II und III von 1:0,7 bis 1,5 arbeitet.

3. Verfahren nach Anspruch 1, **dadurch gekennzeichnet, daß** man mit einem molaren Verhältnis der Verbindungen II und III von 1:0,8 bis 1,3 arbeitet.

4. Verfahren nach Anspruch 1, **dadurch gekennzeichnet, daß** man in Gegenwart eines organischen Lösungsmittels arbeitet.

5. Verfahren nach Anspruch 1, **dadurch gekennzeichnet, daß** man in Gegenwart von Toluol, Xylol, Benzol, Monoglyme, Diglyme, Diisopropylether, Tetrahydrofuran, Dioxan, Isobutylmethylketon, Aceton, Diethylketon, Acetonitril, Carbonsäurealkylester oder deren Gemischen arbeitet.

6. Verfahren nach Anspruch 1, **dadurch gekennzeichnet, daß** man in Carbonsäurealkylestern oder Toluol als Lösungsmittel arbeitet.

7. Verfahren nach Anspruch 1, **dadurch gekennzeichnet, daß** die Menge an Säurehalogenid 0,5 bis 2 Moläquivalente, bezogen auf Amidocarbonsäure II, beträgt.

8. Verfahren nach Anspruch 1, **dadurch gekennzeichnet, daß** die Menge an Säurehalogenid 0,7 bis 1,5 Moläquivalente, bezogen auf Amidocarbonsäure II, beträgt.

9. Verfahren nach Anspruch 1, **dadurch gekennzeichnet, daß** die Menge an Säurehalogenid 0,9 bis 1,4 Moläquivalente, bezogen auf Amidocarbonsäure II, beträgt.

10. Verfahren nach Anspruch 1, **dadurch gekennzeichnet, daß** man bei 25 bis 120°C arbeitet.

## Claims

1. A process for the preparation of amido acid phenyl esters of the formula I 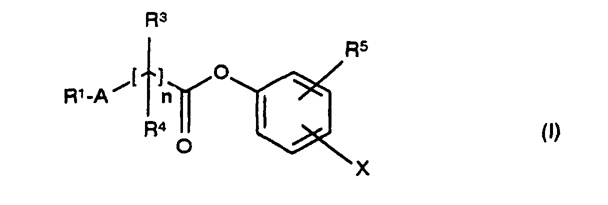 where
A is a group of the formula -CONR²- or -NR²CO-,
R¹ is an alkyl, alkenyl, alkynyl or cycloalkyl group having in each case from 1 to 26 carbon atoms, or an aryl or alkylaryl group having in each case from 6 to 14 carbon atoms,
R² is hydrogen or an alkyl, alkenyl, alkynyl or a cycloalkyl group having in each case from 1 to 26 carbon atoms, or an aryl or alkylaryl group having in each case from 6 to 14 carbon atoms,
R³ and R⁴ may be identical or different and can each be hydrogen or an alkyl, alkenyl, alkynyl or a cycloalkyl group having in each case from 1 to 10 carbon atoms,
R⁵ is hydrogen, halogen or an alkyl, alkenyl, alkynyl, cycloalkyl or an alkoxy group having in each case from 1 to 6 carbon atoms, n is a number from 1 to 10,
X is a group of the formulae SO₃M, OSO₃M, (CH₂)ₘSO₃M, (CH₂)ₘO-SO₃M, CO₂M and N(R⁶)₃Y,
where M is hydrogen or an alkali metal ion,
R⁶ is an alkyl group having from 1 to 6 carbon atoms or a cycloalkyl group having from 4 to 6 carbon atoms,
Y is a halogen atom and
m is 1 or 2,
which comprises adding an inorganic acid halide to a mixture of the compounds of the formulae II and III 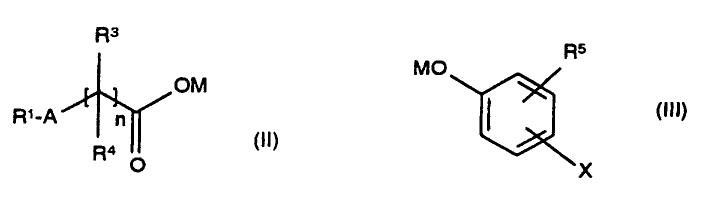 where A, R¹, R², R³, R⁴, R⁵, X and M are as defined above.

2. The process as claimed in claim 1, which is carried out using a molar ratio of the compounds II and III of 1:0.7 to 1.5.

3. The process as claimed in claim 1, which is carried out using a molar ratio of the compounds II and III of 1:0.8 to 1.3.

4. The process as claimed in claim 1, which is carried out in the presence of an organic solvent.

5. The process as claimed in claim 1, which is carried out in the presence of toluene, xylene, benzene, monoglyme, diglyme, diisopropyl ether, tetrahydrofuran, dioxane, isobutyl methyl ketone, acetone, diethyl ketone, acetonitrile, carboxylic alkyl esters or mixtures thereof.

6. The process as claimed in claim 1, which is carried out in carboxylic alkyl esters or toluene as solvent.

7. The process as claimed in claim 1, wherein the amount of acid halide is from 0.5 to 2 molar equivalents, based on amidocarboxylic acid II.

8. The process as claimed in claim 1, wherein the amount of acid halide is from 0.7 to 1.5 molar equivalents, based on amidocarboxylic acid II.

9. The process as claimed in claim 1, wherein the amount of acid halide is from 0.9 to 1.4 molar equivalents, based on amidocarboxylic acid II.

10. The process as claimed in claim 1, which is carried out at from 25 to 120°C.

## Revendications

1. Procédé pour la préparation d'esters phényliques d'acides amidocarboxylique de formule I 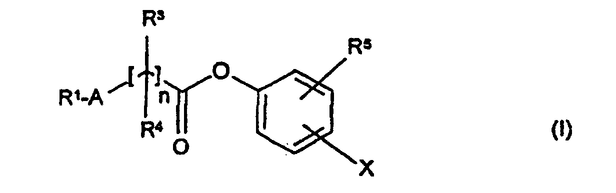 dans laquelle
A représente un groupe de formule -COR²- ou -NR²CO-,
R représente un groupe alkyle, alcényle, alcynyle ou cycloalkyle ayant respectivement de 1 à 26 atomes de carbone ou un groupe aryle ou alkylaryle ayant respectivement de 6 à 14 atomes de carbone,
R² représente un atome d'hydrogène ou un groupe alkyle, alcényle, alcynyle ou cycloalkyle ayant respectivement de 1 à 26 atomes de carbone ou un groupe aryle ou alkylaryle ayant respectivement de 6 à 14 atomes de carbone,
R³ et R⁴ qui peuvent être identiques ou différents, représentent respectivement un atome d'hydrogène ou un groupe alkyle, alcényle, alcynyle ou cycloalkyle ayant respectivement de 1 à 10 atomes de carbone,
R⁵ représente un atome d'hydrogène, d'halogène ou un groupe alkyle, alcényle, alcynyle, cycloalkyle ou un groupe alcoxy ayant respectivement de 1 à 6 atomes de carbone, n représente un nombre de 1 à 10,
X représente un groupe des formules SO₃M, OSO₃M, (CH₂)ₘSO₃M, (CH₂)ₘO-SO₃M, CO₂M et N(R⁶)₃Y,
dans lesquelles M représente un atome d'hydrogène ou un ion alcalin,
R⁶ représente un groupe alkyle ayant de 1 à 6 atomes de carbone ou un groupe cycloalkyle ayant de 4 à 6 atomes de carbone,
Y représente un atome d'halogène et
m représente 1 ou 2,
**caractérisé en ce qu'**on ajoute un halogénure d'acide inorganique à un mélange des composés des formules II et III 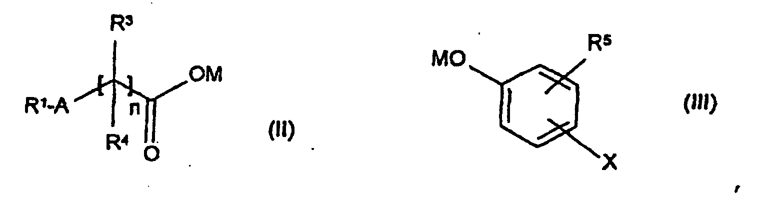 dans lesquelles A, R¹, R², R³, R⁴, R⁵, X et M ont les significations indiquées précédemment.

2. Procédé selon la revendication 1, **caractérisé en ce qu'**on travaille avec un rapport molaire des composés II et III de 1 : 0,7 à 1,5.

3. Procédé selon la revendication 1, **caractérisé en ce qu'**on travaille avec un rapport molaire des composés II et III de 1 : 0,8 à 1,3.

4. Procédé selon la revendication 1, **caractérisé en ce qu'**on travaille en présence d'un solvant organique.

5. Procédé selon la revendication 1, **caractérisé en ce qu'**on travaille en présence de toluène, xylène, benzène, monoglyme, diglyme, diisopropyléther, tétrahydrofurane, dioxane, isobutylméthylcétone, acétone, diéthylcétone, acétonitrile, ester alkylique d'acide carboxylique ou leurs mélanges.

6. Procédé selon la revendication 1, **caractérisé en ce qu'**on travaille dans des esters alkyliques d'acides carboxyliques ou le toluène comme solvant.

7. Procédé selon la revendication 1, caractérisé en que la quantité d'halogénure d'acide est de 0,5 à 2 équivalents molaires, par rapport à l'acide amidocarboxylique II.

8. Procédé selon la revendication 1, caractérisé en que la quantité d'halogénure d'acide est de 0,7 à 1,5 équivalents molaires, par rapport à l'acide amidocarboxylique II.

9. Procédé selon la revendication 1, **caractérisé en ce que** la quantité d'halogénure d'acide est de 0,9 à 1,4 équivalents molaires, par rapport à l'acide amidocarboxylique II.

10. Procédé selon la revendication 1, **caractérisé en ce qu'**on travaille de 25 à 120°C.
